# EUROPEAN PATENT APPLICATION

(11) **EP 0 585 885 A2**
(43) Date of publication of application: **09.03.1994**
(21) Application number: 93113924.0
(22) Date of filing: 31.08.1993
(51) Int. Cl.: C08B 37/08, A61K 7/48

(54) **Hydroxyalkylchitosan salt, production process thereof and cosmetic composition containing the same**

(30) Priority: 01.09.1992 JP 255401/92
(71) Applicant: DAINICHISEIKA COLOR & CHEMICALS MFG. CO. LTD., Chuo-ku Tokyo 103 (JP)
(72) Inventor: Sannan, Takanori, Suginami-ku, Tokyo (JP); Tsuchida, Shinya, Saitama-ken (JP); Ise, Hiroshi, Tokyo (JP); Horiguchi, Shojiro, Saitama-ken (JP)
(74) Representative: Wächtershäuser, Günter, Prof. Dr.

(57) **Abstract**

Described herein are a hydroxyalkylchitosan salt formed from a hydroxyalkylchitosan and an organic or inorganic acid; a production process of the hydroxyalkylchitosan salt, which comprises adding and dissolving a hydroxyalkylchitosan and an acid in water or an aqueous solvent and then allowing the hydroxyalkylchitosan and the acid to undergo a salt-forming reaction; and a cosmetic composition containing as an essential ingredient a water, alcohol or water-alcohol solution of the hydroxyalkylchitosan salt.

## Description

### BACKGROUND OF THE INVENTION

### 1) Field of the Invention

The present invention relates to hydroxyalkylchitosan salts, a production process thereof and cosmetic compositions containing the same. More specifically, this invention is concerned with hydroxyalkylchitosan salts which, when formed into a solution, can provide the solution with excellent long-lasting clarity and are useful as raw materials for cosmetics and the like, a production process thereof and cosmetic compositions containing the same.

### 2) Description of the Related Art

Hydroxypropylated chitosan, which is a kind of hydroxyalkylated deacetylated chitin, is disclosed, for example, in Japanese Patent Publication No. 005601/ 1989. This publication also discloses that hydroxypropylated chitosan is useful for various applications as a water-soluble polymer.

Although such hydroxyalkylated chitosans are water-soluble, the resulting aqueous solutions have a cloud point at 30-50°C. Namely, aqueous solutions of these hydroxyalkylated chitosans are accompanied with the problem in thermal stability that they precipitate from the aqueous solvent at the above temperature and the aqueous solutions hence lose clarity. They are accordingly not suitable as raw materials for cosmetics.

In addition, aqueous solutions of the above-described hydroxyalkylated chitosans have high clarity immediately after preparation. Their clarity, however, drops along the passage of time. Their use as raw materials for cosmetics or food additives therefore involves potential problems.

### SUMMARY OF THE INVENTION

Accordingly, an object of the present invention is to provide a chitosan derivative which can provide an aqueous solution having no cloud point, excellent thermal stability and capable of maintaining high clarity over a long period.

The above object will be achieved by the present invention to be described hereinafter. The present invention therefore provides a hydroxyalkylchitosan salt formed from a hydroxyalkylchitosan and an organic or inorganic acid; a production process of the hydroxyalkylchitosan salt, which comprises adding and dissolving the hydroxyalkylchitosan and the acid in water or an aqueous solvent to allow them to undergo a salt-forming reaction; and a cosmetic composition comprising as an essential ingredient a water, alcohol or water-alcohol solution of the hydroxyalkylchitosan salt.

The salt-forming conversion of the hydroxyalkylchitosan with the organic or inorganic acid has made it possible to provide a chitosan derivative which, when dissolved in an aqueous, alcohol or water-alcohol solution, can provide a solution which has no cloud point, is equipped with excellent heat stability and is capable of maintaining high clarity over a long period.

The chitosan derivative so obtained is superior in compatibility with the hair or the skin to the corresponding hydroxyalkylchitosan itself because the amino groups of the hydroxyalkylchitosan become cationic in the derivative. It has suitable moisturizing property, while retaining the inherent property of the hydroxyalkylchitosan that the hair can be imparted with softness and flexibility. It is therefore useful, for example, as a cosmetic material.

### DETAILED DESCRIPTION OF THE INVENTION AND PREFERRED EMBODIMENTS

The present invention will hereinafter be described more specifically by some preferred embodiments.

Deacetylated chitin useful as a starting material for the preparation of the dehydroxyalkylchitosan employed in the present invention is a deacetylated derivative of chitin which is a kind of natural high-molecular substance found in shells of crabs, shrimps, lobsters, insects or the like or in mushrooms. It is a basic polysaccharide containing 2-amino-2-deoxy-D-glucose as a structural unit.

Such a deacetylated chitin itself has already been produced on an industrial scale and is available in various grades on the market. Industrially-available chitosan has a deacetylation degree of about 70-80% so that it can be used, as is, in the present invention. Particularly preferred is chitosan having a deacetylation degree of at least 90%.

A chitosan having a deacetylation degree of at least 90% can be obtained by, as specifically described in Japanese Patent Publication Nos. 34201/1981 and 00441/1983, subjecting chitin to deacetylation treatment in a concentrated alkaline solution, removing the alkaline solution, washing the deacetylated chitin with water and then repeating these procedures. Particularly preferred is a completely deacetylated chitosan having a deacetylation degree of 95-100%.

To convert chitosan into the hydroxyalkylchitosan, a corresponding alkylene oxide can be used. Examples of the alkylene oxide usable in the present invention include ethylene oxide, propylene oxide and butylene oxide, with the latter two oxides being particularly preferred. It is desired to use the alkylene oxide in an amount of about 5-20 moles per amino group of chitosan. Amounts smaller than 5 moles result in a hydroxyalkylchitosan having insufficient dissolution stability in water. Amounts greater than 20 moles, on the other hand, do not bring about any additional advantages.

Although the above-described hydroxyalkylation of chitosan may be conducted in an aqueous containing an alcoholic organic solvent such as isopropyl alcohol, the hydroxyalkylation can be easily carried out in water free of any organic solvent. The most preferred reaction method is to obtain a target product by suspending chitosan in water, reacting the suspension at 50-100°C for 3-24 hours. During the reaction, chitosan which is distributed as the raw material is converted into a swollen state as the hydroxyalkylation proceeds. It is however necessary to continue the reaction regardless of such swelling.

In a preferred embodiment, chitosan can be highly hydroxyalkylated by adding in the course of the reaction a suitable inorganic acid, for example, a mineral acid such as hydrochloric acid, sulfuric acid or phosphoric acid, or sulfamic acid; an organic acid such as acetic acid, citric acid, adipic acid or pyrrolidonecarboxylic acid; or a mixture thereof to the above-described reaction system which contains swelled chitosan in a dispersed state, thereby adjusting the pH of the resulting mixture to about 2-5 to completely dissolve the reaction mixture in water; and then, continuing the reaction without modifying the reaction system or with the pH of the reaction system being returned from weak acidity to alkalinity.

The molecular weight of the hydroxyalkylchitosan so obtained is preferably 400,000 or lower, with 10,000 - 100,000 being more preferred. The molecular weight can be controlled into the above range by, as disclosed in Japanese Patent Laid-Open No. 40303/1986, subjecting chitosan as the raw material or the resulting hydroxyalkylchitosan to heat treatment in an aqueous solution of an oxidizing agent such as sodium perborate.

If the molecular weight of the hydroxyalkylchitosan exceeds 40,000, the viscosity of its aqueous solution, when formed, is too high to yield any chitosan solution of high concentration. Such a high-molecular hydroxyalkylchitosan is no longer readily usable as a raw material for cosmetics or the like.

No particular limitation is imposed on the acid which is used to convert the hydroxyalkylchitosan into a salt. The acid can be either organic or inorganic. When the hydroxyalkylchitosan salt is used as a raw material for cosmetics, however, it is desired to employ an organic acid which is found in the living body such as lactic acid, pyrrolidonecarboxylic acid or urocanic acid, or which is disclosed in the Cosmetic Material Standards or the Japanese Standards on Food Additives.

To convert the hydroxyalkylchitosan salt into a salt by the above acid, it is necessary to add and dissolve the hydroxyalkylchitosan and the acid in water or an aqueous solvent and then to allow them to undergo a salt-forming reaction. The acid is preferably used in an amount sufficient to maintain the pH of the salt-forming reaction mixture at about 3-7. As the medium of the salt-forming reaction mixture, an aqueous solvent such as water, an alcohol or a water-alcohol mixed solvent is preferred. The concentration of the reaction mixture may be about 10-95 wt.%.

The cosmetic composition of the present invention can be obtained by dissolving the hydroxyalkylchitosan salt of the present invention in water, a mixed solvent of water and an organic solvent such as an alcohol or an alcohol and adding and mixing with the resultant solution a perfume, a colorant, a surface active agent and/or other secondary components as needed. The preferred concentration of the hydroxyalkylchitosan salt is 0.5-5 wt.%. The cosmetic composition according to the present invention is in the form of a liquid or a cream and is therefore suitable for the treatment of human hairs. When sprayed, the composition can impart an excellent moisturized feeling and smoothness to the hair without oiliness while assuring that the hair can be combed through smoothly.

The present invention will hereinafter be described more specifically by the following Referential Examples and Examples, in which all designations of "part" or "parts" mean part or parts by weight unless otherwise specifically indicated.

### Referential Example 1

Charged in an autoclave were 50 parts of chitosan (deacetylation degree: 95%, average molecular weight: 100,000), 300 parts of water, 6 parts of a 20% aqueous sodium hydroxide solution and 300 parts of propylene oxide, followed by reaction at 70°C for 20 hours under stirring. The reaction mixture was cooled and its pH was adjusted to 8-9. The resulting mixture was added dropwise to 5 ℓ of hot water of 90°C under stirring so that the target hydroxypropylchitosan was caused to precipitate. The precipitate was washed with 5 ℓ of hot water, dried at 50°C and then pulverized, whereby 81 parts of hydroxypropylchitosan were obtained in the form of powder. To observe the clarity of its solution, the resulting hydroxypropylchitosan powder was dissolved to a concentration of 5% in 95% ethanol. As a result, the solution retained good clarity over a long period.

### Referential Example 2

Charged in an autoclave were 50 parts of chitosan (deacetylation degree: 95%, average molecular weight: 40,000), 150 parts of water and 100 parts of propylene oxide, followed by gradual heating to 100°C under stirring. The reaction was continued as was for 8 hours, which resulted in a pressure reduction from 5 kg/cm₂ at the beginning to 0.5 kg/cm₂ at the completion of the reaction.

After cooling, 20 parts of pyrrolidonecarboxylic acid (PCA) were added to the reaction mixture at room temperature, followed by stirring for 4 hours so that the reaction mixture was dissolved. A 20% aqueous sodium hydroxide solution (37 parts) was added dropwise to the resulting solution for neutralization. Propylene oxide (200 parts) was added to the neutralized solution, followed by reaction at 70°C for 8 hours. After the reaction mixture was cooled, its pH was adjusted to 8-9. The resulting mixture was added dropwise to 5 ℓ of hot water of 90°C under stirring so that the target hydroxypropylchitosan was caused to precipitate. The precipitate was washed further with 5 ℓ of hot water, dried at 50°C, and then pulverized, whereby 80 parts of hydroxypropylchitosan were obtained in the form of powder.

To observe the clarity of its solution, the resulting hydroxypropyl chitosan powder was dissolved in water to a concentration of 5%. As a result, the solution retained excellent clarity over a long period.

### Referential Examples 3-6

In a similar manner to Referential Example 1 or 2 except that the reaction conditions were changed as shown in Tables 1 and 2, hydroxypropylchitosan was obtained in the form of powder in each Referential Example. Its solution retained good clarity over a long period.

**Table 1**

| | Referential Example 1 | Referential Example 2 | Referential Example 3 |
|---|---|---|---|
| Chitosan | 50 parts | 50 parts | 50 parts |
| Deacetylation degree | 95% | 95% | 95% |
| Average molecular weight | 100,000 | 40,000 | 40,000 |
| Water | 300 parts | 150 parts | 150 parts |
| Propylene oxide | - | 100 parts | 100 parts |
| Butylene oxide | - | - | - |
| Reaction I | - | 100°C,8 hrs | 70°C,20 hrs |
| PCA | - | 20 parts | - |
| Acetic acid | - | - | - |
| Amount of 20% NaOH acid | 6 parts | 37 parts | 6 parts |
| | - | 1.2 moles | - |
| Propylene oxide | 300 parts | 200 parts | 200 parts |
| Butylene oxide | - | - | - |
| Reaction II | 70°C,20 hrs | 70°C,8 hrs | 70°C,8 hrs |
| Yield | 81 parts | 80 parts | 82 parts |
| Long-lasting clarity of the solution | Good | Excellent | Good |

**Table 2**

| | Referential Example 4 | Referential Example 5 | Referential Example 6 |
|---|---|---|---|
| Chitosan | 50 parts | 50 parts | 50 parts |
| Deacetylation degree | 90% | 98% | 95% |
| Average molecular weight | 100,000 | 200,000 | 40,000 |
| Water | 300 parts | 400 parts | 150 parts |
| Propylene oxide | 100 parts | - | 50 parts |
| Butylene oxide | - | 100 parts | 50 parts |
| Reaction I | 100°C,8 hrs | 70°C,20 hrs | 70°C,20 hrs |
| PCA | - | 20 parts | 20 parts |
| Acetic acid | 10 parts | - | - |
| Amount of 20% NaOH acid | 31.3 parts | 29.5 parts | 37 parts |
| | 1.0 mole | 0.95 mole | 1.2 mole |
| Propylene oxide | - | - | 100 parts |
| Butylene oxide | 150 parts | 200 parts | 100 parts |
| Reaction II | 70°C,15 hrs | 70°C,15 hrs | 70°C,8 hrs |
| Yield | 78 parts | 86 parts | 88 parts |
| Long lasting clarity of the solution | Excellent | Excellent | Excellent |

### Examples 1-3

In each Example, 4 parts of the hydroxyalkylchitosan obtained in Referential Example 1, 2 or 3 were dissolved in 96 parts of purified water to obtain an aqueous solution. That aqueous solution was divided into two equal portions, one being as was (Solution A) and the other added with one part of pyrrolidonecarboxylic acid and then, stirred to yield an aqueous solution of the hydroxyalkylchitosan salt of pyrrolidonecarboxylic acid (Solution B). Thus, three kinds of aqueous solutions of the hydroxyalkylchitosans and three kinds of aqueous solutions of the corresponding hydroxyalkylchitosan salts were obtained all together.

Solutions A and B were separately cast on a glass plate, followed by drying under reduced pressure. According to an IR comparison between the resulting films, the existence of the hydroxyalkylchitosan salt of pyrrolidonecarboxylic acid in the film obtained from Solution B was confirmed.

The resulting films were both placed in a vacuum drier at the same time, followed by drying at 50°C for 24 hours. One gram portions of the films were then weighed as quickly as possible and placed on the right and left pans of an balance, respectively. They were left over in a room of 60% humidity and observed. Eight hours later, the pan loaded with the film made from Solution B became lower than the other pan, suggesting that the hydroxyalkylchitosan salt of pyrrolidonecarboxylic acid has higher hygroscopicity than the hydroxyalkylchitosan itself.

When Solution B was heated to its boiling point, no precipitate was observed at any temperature and the solution remained clear.

When Solution A was heated, on the other hand, the solution became turbid at about 48°C and some precipitates were observed.

### Examples 4-6

In each Example, 4 parts of the hydroxyalkylchitosan obtained in Referential Examples 4, 5 or 6 were dissolved in 96 parts of purified water, whereby an aqueous solution was obtained. That aqueous solution was divided into two equal portions, one being as was (Solution C) and the other added with two parts of 50% lactic acid and then stirred to yield an aqueous solution of hydroxyalkylchitosan lactate (Solution D).

Solutions C and D were separately cast on a glass plate, followed by drying under reduced pressure. According to an IR comparison between the resulting films, the existence of the lactate of hydroxyalkylchitosan in the film obtained from solution D was observed. When Solution D was heated to its boiling point, no precipitate was observed at any temperature and the solution remained clear.

Solution C, on the other hand, was somewhat turbid even at room temperature. When it was heated up, precipitates were observed at about 30°C.

### Example 7

In a similar manner to Examples 1-3 except that urocanic acid was used instead of pyrrolidonecarboxylic acid, the results were as in Examples 1-3.

### Example 8

Aqueous solutions of the hydroxyalkylchitosan salts of the corresponding acids obtained in Examples 1-3, respectively, were separately freeze-dried to obtain the salts in the form of solids.

Using each solid salt and its corresponding hydroxyalkylchitosan, separately, shampoos were prepared by way of trial according to the following formulation:

| | |
|---|---|
| Cocoamide propylbetaine (35%) (surface active agent) | 40 parts |
| Water | 54 parts |
| Hydroxyalkylchitosan or its salt | 2 parts |
| "Glucamate DOE-120" (surface active agent) | 3 parts |
| Methylparaben (preservative) | 1 part |

The shampoo of the above formulation with the hydroxyalkylchitosan contained in the form of the salt did not suffer any appreciable loss of clarity even when stored at 40°C over a long period. Hair washed with the shampoo was combed through smoothly, indicating that the shampoo offers excellent conditioning effects.

The shampoo containing the free hydroxyalkylchitosan which had not been converted to its salt, on the other hand, exhibited reduced clarity when stored at 40°C over a long period.

When washed with a shampoo containing the hydroxyalkylchitosan salt of the present invention, the hair, immediately after washing and drying, can be combed smoothly and has an excellent moisturized feeling, compared with a shampoo containing the corresponding hydroxyalkylchitosan. This may be attributable to the reasoning that the hydroxyalkylchitosan salt has better compatibility with the hair protein owing to its cationic property as opposed to the nonionic property of the corresponding hydroxyalkylchitosan, and that more of the free hydroxyalkylchitosan remains on the hair even after thorough post-shampoo rinsing and hence shows excellent moisturization and imparts superb softness and flexibility.

## Claims

1. A hydroxyalkylchitosan salt formed from a hydroxyalkylchitosan and an organic or inorganic acid.

2. A salt of claim 1, wherein the hydroxyalkylchitosan is a reaction product between chitosan and propylene oxide and/or butylene oxide.

3. A salt of claim 1, wherein the organic acid is an acid selected from the group consisting of acids which exist in the living body such as lactic acid, pyrrolidonecarboxylic acid and urocanic acid; those specified in the Cosmetic Material Standards; and those specified in the Japanese Standards on Food Additives.

4. A process for the production of a hydroxyalkylchitosan salt, which comprises adding and dissolving a hydroxyalkylchitosan and an acid in water or an aqueous solvent and then allowing the hydroxyalkylchitosan and the acid to undergo a salt-forming reaction.

5. A process of claim 4, wherein the aqueous solvent is a water-alcohol mixed solvent.

6. A cosmetic composition comprising as an essential ingredient an aqueous, alcohol or water-alcohol solution of a hydroxyalkylchitosan salt.

7. A cosmetic composition of claim 6, wherein the hydroxyalkylchitosan salt is that recited in any one of claims 1-3.

8. A cosmetic composition of claim 7, further comprising a surface active agent.

9. A cosmetic composition of claim 7, which is in a creamy form.
